# EUROPÄISCHE PATENTSCHRIFT

(11) **EP 1 048 654 B1**
(45) Veröffentlichungstag und Bekanntmachung des Hinweises auf die Patenterteilung: **30.07.2003**
(21) Anmeldenummer: 00104753.9
(22) Anmeldetag: 04.03.2000
(51) Int. Cl.: C07D 213/71, C07B 39/00

(54) **Verfahren zur Herstellung von chlorierten Pyridinsulfonsäurechloriden**
Process for the preparation of chloropyridine sulfonyl chloride
Procédé de préparation de chlorure de chloropyridylsulfonyle

(30) Priorität: 21.04.1999 DE 19918021
(43) Veröffentlichungstag der Anmeldung: 02.11.2000
(73) Patentinhaber: Rütgers Organics GmbH, 68305 Mannheim (DE)
(72) Erfinder: Biedenbach, Bruno, 67547 Worms (DE); Michel, Hans-Peter, 68642 Bürstadt (DE)
(74) Vertreter: Grussdorf, Jürgen, Dr.

(56) Entgegenhaltungen:
- DE-A- 1 795 361
- HOUBEN-WEYL, BD 9, 4.AUFL.: "Methoden der Organischen Chemie: Schwefel-, Selen-, Tellurverbindungen" 1955 , GEORG THIEME VERLAG , STUTTGART XP002146358 * Seite 565, Absatz 3 * * Seite 565, Absatz 11 *
- DELARGE, J.: "Chemistry and Pharmacological Properties of the Pyridine-3-sulfonylurea Derivative Torasemide" ARZNEIM.-FORSCHUNG/DRUG RES., Bd. 38, Nr. 1, 1988, Seiten 144-150, XP002146357
- DELARGE, J.: "Synthèse de nouvelles substance antiinflammatoires non stéroïdiques" MEM. ACAD. R. MED. BELG. , Bd. 47, Nr. 3, 1974, Seiten 131-210, XP002146356
- HOUBEN-WEYL, BD. 5 (3), 4. AUFL.: "Methoden der Organischen Chemie: Herstellung von Chlorverbindungen" 1955 , GEORG THIEME VERLAG , STUTTGART XP002146359 * Seite 901, Absatz 2 * * Seite 898, Absatz 4 *

## Beschreibung

Die vorliegende Erfindung betrifft ein neues Verfahren zur Herstellung von Chlorpyridinsulfonsäurechloriden.

Chlorpyridinsulfonsäurederivate dienen als Ausgangsmaterial für die Herstellung einer Reihe von Pharmazeutika mit antiiflammatorischen, antipyretischen, kardiovaskulären, blutzuckersenkenden oder diuretischen Eigenschaften, so beispielsweise 4-Chlorpyridin-3-sulfonamid für die Herstellung von Torasemid und damit verwandten blutzuckersenkenden Sulfonylharnstoffen, die auch entzündungshemmend und diuretisch wirken (J. Delarge, Arzneim.-Forsch./Drug. Res. 38 (I), 1988, 144), wobei das Sulfonamid seinerseits aus 4-Chlorpyridin-3-sulfonsäurechlorid hergestellt wird.

Aus der DE 25 14 334 A1 ist ein Verfahren zur Herstellung von chlorierten Pyridin-3-sulfonsäurederivaten beschrieben, wobei ausgehend von 4-Pyridinol-3-sulfonsäure zunächst die Chlorierung mittels eines Gemisches aus PCl₅ und POCl₃ erfolgt. Das überschüssige POCl₃ und PCl₅ wird im Vakuum abgezogen und der verbleibende Rückstand in mehreren Schritten kompliziert aufgearbeitet. Ähnliche Verfahren unter der Verwendung von PCl₅/POCl₃ sind aus der Literatur bekannt (L. Thunus, Annales pharmaceutiques francaises, 33, 1975, 487; Tullio et. al, Tetrahedron 51, 1995, 3221, sowie EP 0 618 209 A1, DE 25 14 334 und FR 88.352, Delarge, J., Arzneim.-Foschung/Drug Res., Bd. 38, Nr. 1,1988, 144-150 und Delarge, J., MEM. ACAD. R. MED. BELG., Bd. 47, Nr. 3, 1974, 131-210).

Die Chlorierung erfolgt dabei in einem Schritt durch die Substitution der OH-Gruppen am Ring und im Sulfonsäurerest unter Bildung von Chlorwasserstoff und POCl₃. Als Halogenierungsmittel dient ausschließlich das stets im Überschuß eingesetzte Phosphorpentachlorid, während Phosphoroxidchlorid als Lösungsmittel eingesetzt wird, da es auch bei der Umsetzung des Phosphorpentachlorids im Reaktionsgemisch anfällt. Phosphoroxidchlorid selbst führt nicht zur Chlorierung. Nach der Reaktion kann es destillativ aus dem Gemisch entfernt und ohne weitere Reinigungsschritte für den nächsten Ansatz wiederverwendet werden.

Andere Chlorierungsmittel, wie beispielsweise Thionychlorid, Sulfurylchlorid oder auch Phosphortrichlorid ersetzen im wesentlichen nur die phenolische OH-Gruppe und sind von daher für die Reaktion nicht geeignet.

Nachteilig an den bekannten Verfahren ist es, daß bei Ansätzen technischer Größenordnung die Ausgangsmaterialen nicht von Beginn an zusammengefügt werden können, da dies zu nicht mehr beherrschbaren Reaktionsverläufen unter heftiger Gasentwicklung führen kann. Daher erfolgt gemäß dem Stand der Technik eine langsame dosierte Zugabe der Säure zu dem siedenden Gemisch aus Phosphorpentachlorid und Phosphoroxidchlorid, sowie gegebenenfalls auch eine Nachdosierung von PCl₅.

Die Dosierung der im festen Zustand vorliegenden Reaktanden (Hydroxypyridinsulfonsäure und PCl₅) führt jedoch zu erheblichen Problemen. Für die Zugabe ergeben sich verschiedene Möglichkeiten. Entweder muß der Reaktor geöffnet werden, wobei die aggressiven Chemikalien HCl und POCl₃ freigesetzt werden oder die Zuführung erfolgt mit Hilfe von Transportmitteln für Feststoffe, wie beispielsweise Transportschnecken. Auch in diesem Fall ergeben sich Probleme, da der Kontakt der Festsubstanzen mit dem siedenden POCl₃ zu nicht mehr transportierbaren Verklebungen und Verbackungen führt.

Da Phophorpentachlorid stets im Überschuß zugesetzt wird, sublimiert nicht umgesetztes PCl₅ bei der Abdestillation des Lösungsmittels (POCl₃) und führt zu Verkrustungen und Verstopfungen der Kühler. Darüber hinaus sind zusätzliche Reaktionsschritte zur Zerstörung des überschüssigen Halogenierungsmittels durch Hydrolyse auf Eis, ggf. Neutralisation mit Alkali und anschließender Extraktion notwendig. Die Ausbeuten der bekannten Verfahren liegen bei ca. 70%.

Aus der DE 17 95 361 A1 ist bekannt Uracyl-5-carbonsäure mit PCl₃/Cl₂ in POCl₃ als Lösungsmittel bei Siedetemperatur zu 2,4-Dichlor-pyrimidin-5-carbonsäurechlorid in guter Ausbeute und Reinheit umzusetzen. Die Umsetzung der entsprechenden Uracyl-6-carbonsäure verläuft dagegen nur in geringer Ausbeute.

Aus Houben-Weyl, Bd. 9, 4. Aufl., Methoden der organischen Chemie, 1955, Georg Thieme Verlag, Stuttgart, ist bekannt, Pyridin-3-sulfonsäure mit PCl₅ in Xylol zu Pyridin-3-sulfochlorid umzusetzen. Für die Herstellung aromatischer Sulfochloride wird ein Ersatz von PCl₅ durch ein Gemisch von PCl₃/Cl₂ beschrieben. Bei Oxyarylsulfonsäuren kann die Oxy-gruppe sowohl phosphoryliert als auch durch Chlor substituiert werden, als weitere Nebenreaktion wird eine Abspaltung der Sulfonsäure unter Kernchlorierung in gewissen Fällen beschrieben.

Aus Houben-Weyl, Bd. 5 (3), 4. Aufl., Methoden der organischen Chemie, 1955, Georg Thieme Verlag, Stuttgart, ist bekannt, PCl₅ aus PCl₃ und Cl₂ herzustellen.

Die Aufgabe der Erfindung bestand daher darin, ein Verfahren zu finden, mit dem sich Chlor-pyridinsulfonsäurechloride technisch einfach und in hoher Ausbeute herstellen lassen.

Die Aufgabe läßt sich überraschenderweise dadurch lösen, daß man die Chlorierung der Hydroxypyridinsulfonsäuren mit Hilfe eines aus der Carbonsäure-Chemie bekannten Phosphortrichlorid-Chlor-Gemisches durchführt (siehe u. a. DE-OS 1 795 361, DE 28 31 777 A1), wobei man erfindungsgemäß
a) in ein Gemisch von Hydroxypyridinsulfonsäure und von Phosphortrichlorid bei Temperaturen von 70 bis 90°C Chlorgas einleitet und wobei man Phosphortrichlorid in einer Menge von 2,0 bis 2,4 Mol und Chlorgas in einer Menge von 2,0 bis 1,9 Mol bezogen auf die Hydroxypyridinsulfonsäure einsetzt;
b) anschließend auf Temperaturen von 100 bis 120°C erwärmt;
c) das gebildete Phosphoroxidchlorid und ggf. überschüssiges Phosportrichlorid destillativ entfernt;
d) den Rückstand mit einem organischen Lösungsmittel aufnimmt;
e) die flüssige Phase zur Gewinnung des chlorierten Pyridinsulfonsäurechlorids im Vakuum destilliert.

Dazu werden zunächst das flüssige Phosphortrichlorid und die Säure vorlegt und das Gemisch unter Rückfluß erhitzt. Anschließend wird Chlor in der Hitze in die Reaktionsmischung eingeleitet, wodurch sowohl eine Substitution der phenolischen OH-Gruppe als auch der Hydroxylgruppe im Säurerest erfolgt. Überraschenderweise tritt dabei keine Kernchlorierung des Pyridins durch das eingesetzte Chlor auf, sondern ausschließlich der Austausch der Hydroxylgruppen.

Man läßt in an sich bekannter Weise nachreagieren und destilliert Phosphoroxidchlorid und ggf. überschüssiges PCl₃ ab. Der Rückstand wird mit einem vorzugsweise halogenfreien Lösungsmittel, wie beispielsweise Toluol oder Methyl-tert.butylether (MTBE) aufgenommen und eventuell filtriert. Ebenso können halogenierte Lösungsmittel, wie beispielsweise Methylenchlorid, Ethylenchlorid oder auch Chloroform eingesetzt werden. Aus umweltschutzrechtlichen Gesichtspunkten wird jedoch der Einsatz von halogenfreien Lösungsmitteln bevorzugt.

Durch den stöchiometrischen bzw. unterschüssigen Einsatz von Chlor verbleibt nahezu kein unverbrauchtes Chlorierungsmittel im Reaktionsgemisch. Das Sulfonsäurechiorid kann somit direkt destillativ aus dem mit dem Lösungsmittel aufgenommen Rückstand in hoher Ausbeute von über 90% und hohem Reinheitsgrad von über 98% (HPLC) erhalten werden.

In einer bevorzugten Ausführungsform des erfindungsgemäßen Verfahrens wird zur weiteren Erhöhung des Reinheitsgrades des Produktes die organische Phase zur Trennung von eventuell verbliebenem POCl₃ mit Wasser ausgerührt, die wässrige Phase abgetrennt und das Lösungsmittel im Vakuum abgezogen.

Es hat sich gezeigt, daß Phosphortrichlorid sowohl in einem Überschuß, als auch bis zu stöchiometrischen Menge eingesetzt werden kann, vorzugsweise erfolgt zur Minimierung des Materialbedarfs die Vorlage eines ca. 0,15 bis 0,2-fachen Überschusses. Das Chlor wird erfindungsgemäß in äquimolaren Mengen bzw. einem geringen Unterschuß eingesetzt. Vorzugsweise wird mit einem ca. 0,02 bis 0,05 Mol%igem Chlor-Unterschuß gearbeitet. in diesem Fall verbleibt kein unverbrauchtes Chlorierungsmittel im Reaktionsgemisch, wodurch sich die Aufarbeitung des Ansatzes nach der Abtrennung von POCl₃ und PCl₃ durch die Möglichkeit der direkten destillativen Gewinnung des Pyridinsulfonsäurechlorids aus dem mit dem organischen Lösungsmittel aufgenommenen Reaktionsgemisch erheblich vereinfacht.

Die Reaktion läuft in einem Gemisch aus Phosphortrichlorid und der Säure ab, wobei im Verlauf der Reaktion Phosphoroxidchlorid gebildet wird, daß als zusätzliches Lösungsmittel dient. Vorteilhaft kann jedoch eine Zugabe von Phosphoroxidchlorid zur Reaktionsmischung zur anfänglichen Verbesserung der Löslichkeit erfolgen. Typischerweise liegen diese Mengen im Bereich von 0,5 bis 5 Mol bezogen auf 1 Mol eingesetzte Säure.

Als Ausgangsmaterial für das erfindungsgemäße Verfahren können beliebige Hydroxypyridinsulfonsäuren dienen. Bevorzugt werden jedoch Hydroxypyridin-3-sulfonsäuren, insbesondere 4-Hydroxypyridin-3-sulfonsäure eingesetzt

Das chlorierte Pyridinsulfonsäurechlorid wird in nahezu quantitativer Ausbeute erhalten.

Mit dem erfindungsgemäßen Verfahren ergibt sich durch den Umgang mit dem flüssigen Phosphortrichlorid und dem gasförmigen Chlor eine wesentlich vereinfachte Verfahrensführung im Vergleich mit der Handhabung des festen und aggressiven PCl₅. Der Ablauf der Reaktion kann sehr einfach über die Menge und Einleitungsgeschwindigkeit des Chlorgases gesteuert werden.

Die nach dem erfindungsgemäßen Verfahren erhaltene organische Phase, welche das chlorierte Pyridinsulfonsäurechlorid enthält kann vorteilhaft ohne Isolierung des Sulfonsäurechlorids zur Herstellung von chlorierten Pyridinsulfonamiden verwendet werden. Dazu wird die organische Phase nach dem Waschvorgang unter Rühren mit Ammoniakwasser vermischt. Der pH-Wert der Mischung soll dabei im neutralen bis schwach basischen Bereich von etwa 7 bis 9,5 liegen.

Da die Löslichkeit des Produktes im Basischen steigt und dadurch Ausbeuteverluste auftreten können wird nach Beendigung der Reaktion, bei der ein leichter Ammoniaküberschuß vorliegt ggf. mit Säure, wie Salzsäure oder sauren Salzen der pH-Wert der Lösung auf 7 bis 9,5 eingestellt.

Zum Erhalt anderer Säureamide können wässrige Lösungen der entsprechenden Amine eingesetzt werden.

Nach der Reaktion wird das in der wässrigen und organischen Phase schwerlösliche Produkt abfiltriert, mit Wasser chloridfrei und anschließend nochmals mit organischem Lösungsmittel gewaschen. Die Trocknung des gebildeten Amids erfolgt bei Temperaturen von unter 50°C, vorzugsweise bei Raumtemperatur, da das Produkt insbesondere im feuchten Zustand thermolabil ist. Das Produkt wird mit einer Ausbeute von etwa 77 bis 85% erhalten.

### Beispiel 1 (Vergleichsbeispiel mit Cl₂-Überschuß)

### 4-Chlorpyridin-3-sulfonsäurechlorid und 4-Chlorpyridin-3-sulfonamid

Die Vorlage aus 1137,5 g (6,49 Mol) 4-Hydroxypyridin-3-sulfonsäure, 3481 g (2085 ml/22,7 Mol) Phosphoroxidchlorid und 2115 g (1345 ml/15,4 Mol) Phosphortrichlorid wird unter Rühren zum Rückfluß erhitzt, wobei die Sumpftemperatur auf ca. 80°C steigt. In ungefähr 3 Stunden werden in das Reaktionsgemisch 1092 g (15,4 Mol) Chlorgas eingeleitet, welches sofort von der Mischung aufgenommen wird. Unter HCI-Entwicklung steigt die Sumpftemperatur auf ca. 100°C. Nach 24 Stunden Rühren unter Rückfluß, wobei die Sumpftemperatur auf 110°C in den Siedebereich des Phosphoroxidchlorids steigt, wird das Gemisch fast klar. 5550 g (3313 ml) Phosphoroxidchlorid werden im Wasserstrahlvakuum abdestilliert und können wiederverwendet werden. Der Rückstand wird bei ca. 40°C mit 5030 g (4000 ml) Ethylenchlorid aufgenommen und unter äußerer Wasserkühlung bei max. 30°C portionsweise mit 2600 ml Wasser versetzt. Nach der Trennung der Phasen wird die organische Phase 2mal mit je 2500 ml Wasser ausgerührt. Durch das Abdestillieren des Lösungsmittels und Trocknen im Vakuum kann das 4-Chlorpyridin-3-sulfonsäurechlorid in praktisch quantitativer Ausbeute isoliert werden.

Technisch wird jedoch zur Herstellung des Sulfonsäureamids die Lösung direkt unter weiterem Rühren und äußerer Kühlung portionsweise mit 1015 g (1120 ml/14,93 Mol) 25%igem Ammoniakwasser vermischt. Dabei soll die Temperatur 30-35°C nicht übersteigen. Man läßt ca. 3 Stunden intensiv nachrühren, wobei der pH-Wert im basischen Bereich verbleiben soll. Anschließend wird mit ca. 195 g (165 ml/1,95 Mol) konzentrierter Salzsäure neutralisiert und nach weiteren 3 Stunden Rühren abfiltriert. Der Rückstand wird mit 3250 ml Wasser und anschließend mit 2075 g (1650 ml) Ethylenchlorid gewaschen und an der Luft bei Raumtemperatur getrocknet.

Man erhält ca. 1050 g (5,45 Mol) 4-Chlorpyridin-3-sulfonamid in ca. 84% Ausbeute. Das Produkt weist einen Schmelzpunkt von 152°C unter Zersetzung und einen Chlorgehalt von 18,1 bis 18,6% auf. Die Reinheit beträgt 99% (HPLC).

Als Lösungsmittel kann auch Chloroform verwendet werden, wobei in diesem Fall aufgrund der geringeren Löslichkeit des 4-Chlorpyridin-3-sulfonsäurechlorids das doppelte Lösungsmittelvolumen benötigt wird.

### Beispiel 2

### 4-Chlorpyridin-3-sulfonsäurechlorid und 4-Chlorpyridin-3-sulfonamid

Die Vorlage aus 1137,5 g (6,49 Mol) 4-Hydroxypyridin-3-sulfonsäure, 3481 g (2085 ml/22,7 Mol) Phosphoroxidchlorid und 2115 g (1345 ml/15,4 Mol) Phosphortrichlorid wird unter Rühren zum Rückfluß erhitzt, wobei die Sumpftemperatur auf ca. 80°C steigt. In ungefähr 3 Stunden werden in das Reaktionsgemisch 920 g (12,98 Mol) Chlorgas eingeleitet, welches sofort von der Mischung aufgenommen wird. Unter HCl-Entwicklung steigt die Sumpftemperatur auf ca. 100°C. Nach 24 Stunden Rühren unter Rückfluß, wobei die Sumpftemperatur auf 110°C in den Siedebereich des Phosphoroxidchlorids steigt, wird das Gemisch fast klar. 5550 g (3313 ml) Phosphoroxidchlorid werden im Wasserstrahlvakuum abdestilliert und können wiederverwendet werden. Der Rückstand wird bei ca. 40°C mit 5030 g (4000 ml) Ethylenchlorid aufgenommen und unter äußerer Wasserkühlung bei max. 30°C portionsweise mit 2600 ml Wasser versetzt. Nach der Trennung der Phasen wird die organische Phase 2mal mit je 2500 ml Wasser ausgerührt. Durch das Abdestillieren des Lösungsmittels und Trocknen im Vakuum kann das 4-Chlorpyridin-3-sulfonsäurechlorid in praktisch quantitativer Ausbeute isoliert werden.

Technisch wird jedoch zur Herstellung des Sulfonsäureamids die Lösung direkt unter weiterem Rühren und äußerer Kühlung portionsweise mit 1015 g (1120 ml/14,93 Mol) 25%igem Ammoniakwasser vermischt. Dabei soll die Temperatur 30-35°C nicht übersteigen. Man läßt ca. 3 Stunden intensiv nachrühren, wobei der pH-Wert im basischen Bereich verbleiben soll. Anschließend wird mit ca. 195 g (165 ml/1,95 Mol) konzentrierter Salzsäure neutralisiert und nach weiteren 3 Stunden Rühren abfiltriert. Der Rückstand wird mit 3250 ml Wasser und anschließend mit 2075 g (1650 ml) Ethylenchlorid gewaschen und an der Luft bei Raumtemperatur getrocknet.

Man erhält ca. 1050 g (5,45 Mol) 4-Chlorpyridin-3-sulfonamid in ca. 84% Ausbeute. Das Produkt weist einen Schmelzpunkt von 153°C unter Zersetzung auf. Die Reinheit beträgt 99,6% (HPLC).

Als Lösungsmittel kann auch Chloroform verwendet werden, wobei in diesem Fall aufgrund der geringeren Löslichkeit des 4-Chlorpyridin-3-sulfonsäurechlorids das doppelte Lösungsmittelvolumen benötigt wird.

### Beispiel 3

### 4-Chlorpyridin-3-sulfonamid

Es werden 25 g (0,16 mol) Phosphoroxidchlorid vorgelegt und 103 g (0,75 mol) Phosphortrichlorid zugesetzt. Unter Rühren werden zur Lösung 56,3 g (0,32 mol) 4-Hydroxypyridin-3-sulfonsäure gegeben und auf ca. 80 °C zum Rückfluß erhitzt. Dann werden 44,5 g (0,62 mol) Chlor innerhalb von 3 bis 4 Stunden eingeleitet, wobei die Rückflußtemperatur auf 103 bis 108°C steigt. Man läßt 20 Stunden unter Rühren nachreagieren, wobei die Temperatur auf 105 bis 110°C steigt und die gelbliche Lösung fast klar wird. Dann wird auf ca. 50°C abgekühlt und Phosphoroxidchiorid und PCl₃ unter Vakuum vollständig abdestilliert. Der Destillationsrückstand wird mit 200 ml Toluol versetzt, bei 20 bis 25°C abfiltriert und der Filterrückstand nochmals mit wenig Toluol gewaschen. Die organische Phase wird 2mal mit jeweils 100 ml Wasser ausgerührt und die wässrige Phase abgetrennt. Die toluolische Phase wird mit 30 ml Aceton gemischt und 52 bis 55 ml (0,67 - 0,7 mol) einer 24%igen wässrigen Ammoniaklösung bei 20 bis 25°C unter Rühren innerhalb von ca. 3 Stunden zugetropft. Weitere 9 Stunden wird nachgerührt, wobei der pH-Wert der Lösung am Ende der Reaktion bei ca. 9 liegt. Das ausgefallene Produkt wird abgesaugt und 3mal mit jeweils 50 ml Wasser chloridfrei und anschließend 2mal mit je 50 ml Toluol gewaschen. Das Produkt wird bei Raumtemperatur getrocknet. Man erhält 51,6 g (0,268 mol) 4-Chlorpyridin-3-sulfonamid mit einem Schmelzpunkt von 153°C (Zersetzung), entsprechend einer Ausbeute von ca. 84%. Die Reinheit beträgt 99,7% (HPLC).

Die Zugabe von Aceton (vorzugsweise 15% bezogen auf den Toluoleinsatz) ist für die Reinheit und Ausbeute unerheblich. Durch den Zusatz wird einerseits ein Anbacken des Produkts an der Reaktorwandung verhindert und andererseits eine bessere Durchmischung der Phasen und damit letztlich eine Beschleunigung der Reaktion erreicht.

Anstelle von Toluol kann auch MTBE als Lösungsmittel eingesetzt werden.

### Beispiel 4

### 4-Chlorpyridin-3-sulfonamid

Es werden 137 g Phosphortrichlorid vorgelegt. Unter Rühren werden zur Lösung 56,3 g (0,32 mol) 4-Hydroxypyridin-3-sulfonsäure gegeben und auf ca. 77 °C zum Rückfluß erhitzt. Dann werden 44,5 g (0,62 mol) Chlor innerhalb von 3 bis 4 Stunden eingeleitet, wobei die Rückflußtemperatur nach und nach ansteigt. Man läßt 20 Stunden unter Rühren nachreagieren, wobei die Temperatur auf 105 bis 110°C steigt und die gelbliche Lösung fast klar wird. Dann wird auf ca. 50°C abgekühlt und Phosphoroxidchlorid und PCl₃ unter Vakuum vollständig abdestilliert. Der Destillationsrückstand wird mit 200 ml Toluol versetzt, bei 20 bis 25°C abfiltriert und der Filterrückstand nochmals mit wenig Toluol gewaschen. Die organische Phase wird 2mal mit jeweils 100 ml Wasser ausgerührt und die wässrige Phase abgetrennt. Die toluolische Phase wird mit 30 ml Aceton gemischt und 52 bis 55 ml (0,67 - 0,7 mol) einer 24%igen wässrigen Ammoniaklösung bei 20 bis 25°C unter Rühren innerhalb von ca. 3 Stunden zugetropft. Weitere 9 Stunden wird nachgerührt, wobei der pH-Wert der Lösung am Ende der Reaktion bei ca. 9 liegt. Das ausgefallene Produkt wird abgesaugt und 3mal mit jeweils 50 ml Wasser chloridfrei und anschließend 2mal mit je 50 ml Toluol gewaschen. Das Produkt wird bei Raumtemperatur getrocknet. Man erhält 51,1 g (0,265 mol) 4-Chlorpyridin-3-sulfonamid mit einem Schmelzpunkt von 153°C (Zersetzung), entsprechend einer Ausbeute von ca. 83%. Die Reinheit beträgt 99,7% (HPLC).

Die Zugabe von Aceton (vorzugsweise 15% bezogen auf den Toluoleinsatz) ist für die Reinheit und Ausbeute unerheblich. Durch den Zusatz wird einerseits ein Anbacken des Produkts an der Reaktorwandung verhindert und andererseits eine bessere Durchmischung der Phasen und damit letztlich eine Beschleunigung der Reaktion erreicht.

Anstelle von Toluol kann auch MTBE als Lösungsmittel eingesetzt werden.

### Beispiel 5

### 4-Chlorpyridin-3-sulfonsäurechlorid

Wie unter Beispiel 4, jedoch werden 150 g (0,98 mol) Phosphoroxidchlorid, 618 g (4,5 mol) Phosphortrichlorid und 336 g (1,92 mol) 4-Hydroxypyridin-3-sulfonsäure vorgelegt und 267 g (3,75 mol) Chlor im Unterschuß innerhalb von 6 Stunden eingeleitet. Nach Zugabe von 1200 ml Toluol und Ausrühren mit 2mal 300 ml Wasser wird die organische Phase abgetrennt und das Toluol im Vakuum entfernt. Das Produkt wird bei einer Kopftemperatur von 84 bis 105°C und 0,1 bis 0,2 mmHg im Vakuum überdestilliert. Man erhält 372 g (1,75 mol) 4-Chlorpyridin-3-sulfonsäurechlorid, entsprechend einer Ausbeute von 93,3% bezogen auf das eingesetzte Chlor und von 91,4% bezogen auf die vorgelegte 4-Hydroxypyridin-3-sulfonsäure.

Das erhaltene 4-Chlorpyridin-3-sulfonsäurechlorid weist einen Schmelzpunkt von 44°C (farblose Schmelze) auf. Das ¹H-NMR-Spekrum (CDCl₃) zeigt folgende Strukturen: 7,70 (d, 1H), 8,80 (d, 1H) und 9,20 (s, 1H). Die Reinheit beträgt 99,8% (HPLC).

Das Produkt kann gegebenenfalls auch direkt nach Abdestillation von PCl₃ und POCl₃ im Vakuum destilliert werden.

### Beispiel 6

### 4-Chlorpyridin-3-sulfonsäurechlorid

Wie unter Beispiel 4, jedoch werden 893 g (6,5 mol) Phosphortrichlorid und 336 g (1,92 mol) 4-Hydroxypyridin-3-sulfonsäure vorgelegt und 267 g (3,75 mol) Chlor im Unterschuß innerhalb von 6 Stunden eingeleitet. Nach der Zugabe von 1200 ml Toluol und Ausrühren mit 2mal 300 ml Wasser wird die organische Phase abgetrennt und das Toluol im Vakuum entfernt. Das Produkt wird bei einer Kopftemperatur von 84 bis 105°C und 0,1 bis 0,2 mmHg im Vakuum überdestilliert. Man erhält 366 g (1,72 mol) 4-Chlorpyridin-3-sulfonsäurechlorid, entsprechend einer Ausbeute von 91,8% bezogen auf das eingesetzte Chlor und von 90,0% bezogen auf die vorgelegte 4-Hydroxypyridin-3-sulfonsäure.

Das erhaltene 4-Chlorpyridin-3-sulfonsäurechlorid weist einen Schmelzpunkt von 44°C (farblose Schmelze) auf. Das ¹H-NMR-Spekrum (CDCl₃) zeigt folgende Strukturen: 7,70 (d, 1H), 8,80 (d, 1H) und 9,20 (s, 1H). Die Reinheit beträgt 99,8% (HPLC).

Das Produkt kann gegebenenfalls auch direkt nach Abdestillation von PCl₃ und POCl₃ im Vakuum destilliert werden.

### Beispiel 7

### 4-Chlorpyridin-3-sulfonsäurechlorid

Wie unter Beispiel 3, jedoch werden 150 g (0,98 mol) Phosphoroxidchlorid, 618 g (4,5 mol) Phosphortrichlorid und 336 g (1,92 mol) 4-Hydroxypyridin-3-sulfonsäure vorgelegt und 267 g (3,75 mol) Chlor im Unterschuß innerhalb von 6 Stunden eingeleitet. Nach der Zugabe von 1200 ml Toluol werden die unlöslichen Bestandteile abfiltriert. Das Toluol und restliches Phosphoroxidchlorid werden im Vakuum entfernt. Das Produkt wird bei einer Kopftemperatur von 84 bis 105°C und 0,1 bis 0,2 mmHg im Vakuum überdestilliert. Man erhält 371g (1,75 mol) 4-Chlorpyridin-3-sulfonsäurechlorid, entsprechend einer Ausbeute von 93,0% bezogen auf das eingesetzte Chlor und von 91,1% bezogen auf die vorgelegte 4-Hydroxypyridin-3-sulfonsäure.

Das erhaltene 4-Chlorpyridin-3-sulfonsäurechlorid weist einen Schmelzpunkt von 44°C (farblose Schmelze) auf. Das ¹H-NMR-Spekrum (CDCl₃) zeigt folgende Strukturen: 7,70 (d, 1H), 8,80 (d, 1H) und 9,20 (s, 1H). Die Reinheit beträgt 98,5% (HPLC).

## Patentansprüche

1. Verfahren zur Herstellung von chlorierten Pyridinsulfonsäurechloriden der allgemeinen Formel I 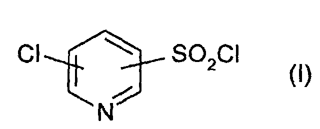 aus Hydroxypyridinsulfonsäuren der allgemeinen Formel II 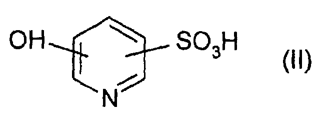 durch die Substitution der ringständigen OH-Gruppe und der OH-Gruppe des Säurerestes durch ein Chlorierungsmittel **dadurch gekennzeichnet, daß** man als Chlorierungsmittel ein Gemisch aus Phosphortrichlorid und Chlorgas verwendet, wobei man
a) in ein Gemisch von Hydroxypyridinsulfonsäure und von Phosphortrichlorid bei Temperaturen von 70 bis 90°C Chlorgas einleitet, wobei man Phosphortrichlorid in einer Menge von 2,0 bis 2,4 Mol pro Mol Hydroxypyridinsulfonsäure und Chlorgas in einer Menge von 2,0 bis 1,9 Mol bezogen auf die Hydroxypyridinsulfonsäure einsetzt;
b) anschließend auf Temperaturen von 100 bis 120°C erwärmt;
c) das gebildete Phosphoroxidchlorid und ggf. überschüssiges Phosportrichlorid destillativ entfernt;
d) den Rückstand mit einem organischen Lösungsmittel aufnimmt;
e) die flüssige Phase zur Gewinnung des chlorierten Pyridinsulfonsäurechlorids im Vakuum destilliert.

2. Verfahren nach Anspruch 1 **dadurch gekennzeichnet, daß** man Phosphortrichlorid im 0,15 bis 0,2-fachen Überschuß und Chlorgas in einem Unterschuß von 0,02 bis 0,05 Mol% einsetzt.

3. Verfahren nach Anspruch 1 oder 2 **dadurch gekennzeichnet, daß** man als organische Lösungsmittel Toluol, MTBE, Chloroform, Methylenchlorid oder Ethylenchlorid verwendet.

4. Verfahren nach einem der Ansprüche 1 bis 3 **dadurch gekennzeichnet, daß** der Reaktionsmischung vor der Chlorierung Phosphoroxidchlorid zugesetzt wird.

5. Verfahren nach einem der vorhergehenden Ansprüche **dadurch gekennzeichnet, daß** man als Hydroxypyridinsulfonsäure 4-Hydroxypyridin-3-sulfonsäure einsetzt.

6. Verfahren nach mindestens einem der Ansprüche 1 bis 5 **dadurch gekennzeichnet, daß** man den mit einem organischen Lösungsmittel aufgenommenen Rückstand der Abdestillation von Phosphoroxidchlorid und Phosphortrichlorid mit Wasser ausrührt, die organische Phase zur Gewinnung des Chlorpyridinsulfonsäurechlorids eindampft und das Produkt im Vakkuum bei 0,1 bis 0,3 mmHg und einer Temperatur von 80 bis 115°C destilliert.

## Claims

1. Process for the preparation of chlorinated pyridinesulphonic acid chlorides of the general formula I 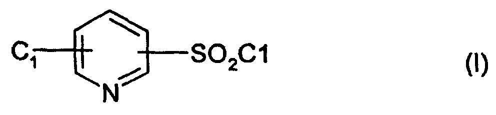 from hydroxypyridine-sulphonic acids of the general formula II 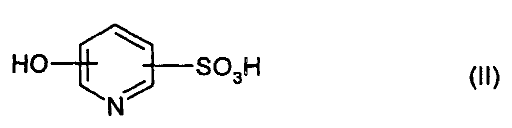 by substitution of the ring OH group and of the OH group of the acid residue by a chlorination agent, **characterised in that** as chlorination agent there is used a mixture of phosphorus trichloride and chlorine gas, whereby
a) into a mixture of hydroxypyridine-sulphonic acid and of phosphorus trichloride, chlorine gas is passed in at a temperature of 70 to 90°C, whereby phosphorus trichloride is used in an amount of 2.0 to 2.4 mol per mol hydroxypyridine sulfonic acid and chlorine gas in an amount of 2.0 to 1.9 mol referred to the hydroxypyridine-sulphonic acid;
b) subsequently, it is heated to temperatures of 100 to 120°C;
c) the phosphorus oxychloride formed and possibly excess phosphorus trichloride are removed distillatively;
d) the residue is taken up with an organic solvent;
e) the liquid phase is distilled in a vacuum for obtaining the chlorinated pyridine-sulphonic acid chloride.

2. Process according to claim 1, **characterised in that** phosphorus trichloride is used in 0.15 to 0.2 fold excess and chlorine gas in an insufficiency of 0.02 to 0.05 mol %.

3. Process according to claim 1 or 2, **characterised in that** toluene, MTBE, chloroform, methylene chloride or ethylene chloride is used as organic solvent.

4. Process according to one of claims 1 to 3, **characterised in that** phosphorus oxychloride is added to the reaction mixture before the chlorination.

5. Process according to one of the preceding claims, **characterised in that** 4-hydroxypyridine-3-sulphonic acid is used as hydroxypyridine sulphonic acid.

6. Process according to at least one of claims 1 to 5, **characterised in that** the residue of the distilling off of phosphorus oxychloride and phosphorus trichloride taken up in an organic solvent is stirred with water, the organic phase is evaporated for obtaining the chloropyridine-sulphonic acid chloride and the product distilled in a vacuum at 0.1 to 0.3 mm Hg and at a temperature of 80 to 115°C.

## Revendications

1. Procédé de préparation de chlorures d'acides pyridinesulfoniques chlorés répondant à la formule générale I 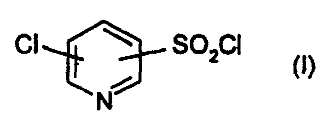 à partir d'acides hydroxypyridinesulfoniques répondant à la formule générale II 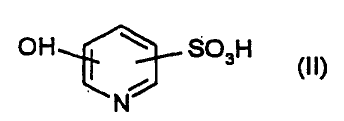 via substitution du groupe nucléaire OH et du groupe OH du radical acide par un agent de chloration, **caractérisé en ce qu'**on utilise, à titre d'agent de chloration, un mélange de trichlorure de phosphore et de chlore gazeux, procédé dans lequel
a) on introduit du chlore gazeux à des températures de 70 à 90 °C dans un mélange d'acide hydroxypyridinesulfonique et de trichlorure de phosphore, en mettant en oeuvre du trichlorure de phosphore en une quantité de 2,0 à 2,4 moles par mole d'acide hydroxypyridinesulfonique et du chlore gazeux en une quantité de 2,0 à 1,9 mole, rapportés à l'acide hydroxypyridinesulfonique ;
b) on chauffe ensuite à des températures de 100 à 120 °C ;
c) on élimine par distillation l'oxychlorure de phosphore obtenu et le cas échéant le trichlorure de phosphore en excès ;
d) on reprend le résidu dans un solvant organique ;
e) on distille la phase gazeuse sous vide pour obtenir le chlorure de l'acide chloropyridinesulfonique.

2. Procédé selon la revendication 1, **caractérisé en ce qu'**on met en oeuvre du trichlorure de phosphore en un excès de 0,15 à 0,2 fois et du chlore gazeux en une quantité insuffisante de 0,02 à 0,05 mole %.

3. Procédé selon la revendication 1 ou 2, **caractérisé en ce qu'**on utilise à titre de solvant organique, du toluène, de l'éther méthyl tert.-butylique (MTBE), du chloroforme, du chlorure de méthylène ou du chlorure d'éthylène.

4. Procédé selon l'une quelconque des revendications 1 à 3, **caractérisé en ce qu'**on ajoute au mélange réactionnel, avant la chloration, de l'oxychlorure de phosphore.

5. Procédé selon l'une quelconque des revendications précédentes, **caractérisé en ce qu'**on met en oeuvre, à titre d'acide hydroxypyridinesulfonique, de l'acide 4-hydroxypyridine-3-sulfonique.

6. Procédé selon au moins une des revendications 1 à 5, **caractérisé en ce qu'**on agite en profondeur avec de l'eau le résidu de la séparation par distillation de l'oxychlorure de phosphore et du trichlorure de phosphore, repris dans un solvant organique, on évapore la phase organique pour obtenir le chlorure de l'acide chloropyridinesulfonique et on soumet le produit à une distillation sous vide sous une pression de 0,1 à 0,3 mmHg et à une température de 80 à 115 °C.
